# EUROPÄISCHE PATENTANMELDUNG

(11) **EP 0 773 213 A2**
(43) Veröffentlichungstag der Anmeldung: **14.05.1997**
(21) Anmeldenummer: 96117259.0
(22) Anmeldetag: 28.10.1996
(51) Int. Cl.: C07C 319/18, C07C 323/56

(54) **Verfahren zur Herstellung von (2RS,3RS)-3-(2'-Aminophenylthio)-2-hydroxy-3-(4"-methoxyphenyl)-propionsäuremethylester**

(30) Priorität: 09.11.1995 DE 19541717
(71) Anmelder: BAYER AG, 51368 Leverkusen (DE)
(72) Erfinder: Hagedorn, Ferdinand, Dr., 51381 Leverkusen (DE); Fiege, Helmut, Dr., 51373 Leverkusen (DE)

(57) **Zusammenfassung**

(2 RS, 3 S)-3-(2'-Aminophenylthio)-2-hydroxy-3-(4''-methoxyphenyl)propionsäuremethylester, ein wichtiges Vorprodukt zur Herstellung des pharmazeutisch wirksamen Stoffes Diltiazem, wird in besonders hoher Stereoselektivität und Ausbeute durch Addition von o-Aminothiophenol an 3-(4'-Methoxyphenyl)-glycidylsäuremethylester erhalten, wenn man die Reaktion bei Anwesenheit von Alkalisalzen schwacher Säuren in Gegenwart katalytischer Mengen von Eisenverbindungen durchführt.

## Beschreibung

Die vorliegende Erfindung betrifft ein verbessertes Verfahren zur Herstellung von (2RS, 3RS)-3-(2'-Aminophenylthio)-2-hydroxy-3-(4''-methoxyphenyl)-propionsäuremethylester (im folgenden auch als AHMPE bezeichnet), durch selektive Addition von o-Aminothiophenol an 3-(4'-Methoxyphenyl)-glycidsäuremethylester (im folgenden auch als MPG-Ester bezeichnet).

Es ist bekannt, durch Addition von unsubstituiertem o-Aminothiophenol an MPG-Ester in einem unpolaren Lösungsmittel und bei erhöhter Temperatur AHMPE herzustellen. AHMPE ist ein Vorprodukt für den Pharma-Wirkstoff Diltiazem (siehe Chem. Pharm. Bull. 18, 2028 (1970)).

Die Herstellung von AHMPE aus MPG-Ester und o-Aminothiophenol verläuft nach folgendem Reaktionsschema:

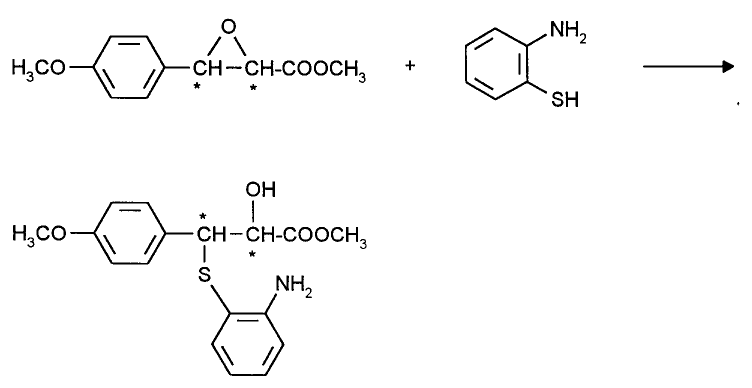

Die EP-OS 609 031 beschreibt ein Verfahren, bei dem die Addition von o-Aminoalkylamino-thiophenol an MPG-Ester zur threo-Form des Ester-Addukts in Gegenwart von zwei- oder dreiwertigen Eisenionen durchgeführt wird.

Frühere Untersuchungen haben allerdings gezeigt, daß bei der Addition von o-Nitrothiophenol an Phenylglycidester zur threo-Form des Ester-Addukts Eisen(II)- und Eisen(III)-chlorid nicht katalytisch wirksam sind. Für diese Reaktion wurden vielmehr Zinnverbindungen als wirksame Katalysatoren gefunden (siehe J. Chem. Soc. Perkin Trans. I, 1984, 1725, insbesondere Tabelle 4 auf Seite 1728).

Eigene Versuche zeigten (siehe Vergleichsbeispiel 2), daß beim Einsatz von hochgereinigtem MPG-Ester bei der Addition von o-Aminothiophenol in Abwesenheit von Eisensalzen eine hohe Stereoselektivität der Reaktion erzielt wird. Die Bereitstellung von hochgereinigtem MPG-Ester erfordert aber erheblichen Aufwand, weil dann z.B. mehrfach Umlösungen und/oder Umkristallisationen vorgenommen werden müssen.

Bei der üblichen Herstellung des MPG-Esters aus Anisaldehyd und Chloressigsäuremethylester in Gegenwart von Natriummethylat mit anschließendem Aufnehmen des rohen MPG-Esters aus dem Reaktionsgemisch mit einem unpolaren Lösungsmittel, z.B. Toluol oder Xylol, verbleiben auch bei intensiver Extraktion der Lösung des rohen MPG-Esters mit Wasser Spuren von Alkalisalzen schwacher Säuren in der organischen Phase (siehe Vergleichsbeispiel 1). Der ohne besonderen Aufwand für die Reinigung zugängliche MPG-Ester enthält Spuren von beispielsweise Natriumacetat und/oder Natrium-3-(4'-methylphenyl)-glycidat.

Es wurde jetzt festgestellt, daß bereits einige ppm Alkalisalze schwacher Säuren bei der Addition von o-Aminothiophenol an den MPG-Ester die Reaktion zum unerwünschten stereoisomeren AHMPE der erythro-Form lenken. Diese schmilzt deutlich niedriger als das gewünschte threo-AHMPE und besitzt ein hohes Lösevermögen für das threo-Produkt. Auf diese Weise kommt es beim Einsatz von wenige ppm Alkalisalze schwacher Säure enthaltendem MPG-Ester zu Ausbeuteverlusten am gewünschten threo-AHMPE in kaum vermuteter Höhe (siehe Beispiele 2 bis 7).

Es wurde nun ein Verfahren zur Herstellung von (2RS, 3RS)-3-(2'-Aminophenylthio)-2-hydroxy-3(4''-methoxyphenyl)-propionsäuremethylester durch Addition von o-Aminothiophenol an 3-(4'-Methoxyphenyl)-glycidylmethylester gefunden, das dadurch gekennzeichnet ist, daß man die Reaktion bei Anwesenheit von Alkalisalzen schwacher Säuren in Gegenwart katalytischer Mengen von Eisenverbindungen durchführt.

In das erfindungsgemäße Verfahren kann man z.B. 1 bis 200 ppm Eisenverbindungen, gerechnet als Eisen und bezogen auf o-Aminothiophenol, einsetzen.

Bevorzugt liegt diese Menge im Bereich 1 bis 100 ppm, insbesondere bei 1 bis 20 ppm. Die optimale Menge Eisenverbindungen richtet sich nach dem Gehalt des eingesetzten MPG-Esters an Alkalisalzen schwacher Säuren und kann gegebenenfalls durch einfache Routineversuche ermittelt werden.

Für das erfindungsgemäße Verfahren eignen sich grundsätzlich alle Verbindungen des Eisens, die im Reaktionsgemisch in der erforderlichen Konzentration löslich sind. Bevorzugt sind zwei- und dreiwertiges Eisen enthaltende Salze, z.B. Eisen(III)-sulfat-pentahydrat, Eisen(II)-sulfat, Eisen(III)-chlorid, Eisen(II)-chlorid und Eisensalze organischer Säuren. Auch komplexe Eisenverbindungen, z.B. Eisenphthalocyanin und Eisenacetylacetonat eignen sich für diesen Zweck. Es können einzelne Eisenverbindungen oder Gemische von mehreren Eisenverbindungen eingesetzt werden.

Die Eisenverbindungen können in fester Form, in Suspension oder in Lösung dem Reaktionsgemisch zugefügt werden. Sie können auch gelöst im umzusetzenden o-Aminothiophenol oder in der Lösung des umzusetzenden MPG-Esters oder in beiden gelöst dem Reaktionsgemisch zugefügt werden. Als Reaktionsmedium zur Durchführung des Verfahrens der vorliegenden Erfindung kommen z.B. unpolare Lösungsmittel in Betracht. Bevorzugt sind Toluol und Xylol.

Man kann bei der Durchführung der Additionsreaktion z.B. so verfahren, daß man o-Aminothiophenol und MPG-Ester zusammen mit der katalytischen Menge Eisenverbindungen in Toluol oder Xylol löst, die Lösung auf Reaktionstemperatur erhitzt und bei dieser Temperatur unter Rühren umsetzt.

Eine andere Methode der Reaktionsdurchführung besteht darin, eine Lösung des MPG-Esters in einem unpolaren Lösungsmittel auf Reaktionstemperatur zu erhitzen und bei dieser Temperatur Eisenverbindungen enthaltendes o-Aminothiophenol in das Reaktionsgemisch einzutragen. Hat man nur eisenfreies o-Aminothiophenol zur Verfügung, dann kann die geringe Menge Eisenverbindungen der Lösung des MPG-Esters bereits auch vor dem Erhitzen zugemischt werden.

Auch eine Verfahrensvariante, bei der man ein Eisenverbindungen enthaltendes o-Aminothiophenol, gegebenenfalls gelöst in einem unpolaren Lösungsmittel, erhitzt und bei Reaktionstemperatur die Lösung des MPG-Esters zufügt, ist geeignet.

Da die Additionsreaktion exotherm verläuft, ist es vorteilhaft, durch Dosieren von jeweils einem der Reaktionspartner die Temperatur des Reaktionsgemisches zu steuern.

Die Reaktion kann beispielsweise bei 80 bis 150°C, vorzugsweise bei 90 bis 120°C durchgeführt werden. Bei zu niedrigen Temperaturen verläuft die Reaktion sehr langsam und wenig selektiv.

Die Reaktionsdauer kann beispielsweise 1 bis 12 Stunden, bevorzugt 2 bis 8 Stunden, besonders bevorzugt 3 bis 5 Stunden betragen. Längere Reaktionszeiten sind im allgemeinen nicht kritisch, weil sich die Zusammensetzung des Reaktionsgemisches nach erfolgter Umsetzung kaum noch ändert.

o-Aminothiophenol oder MPG-Ester kann gegebenenfalls im Überschuß eingesetzt werden, beispielsweise in einer Menge von bis zu 2 mol pro mol des anderen Reaktionspartners. Vorzugsweise arbeitet man bei einem molaren Verhältnis von o-Aminothiophenol zu MPG-Ester von 0,9:1 bis 1,1:1, insbesondere bei stöchiometrischem Mengenverhältnis.

Das Reaktionsgemisch kann beispielsweise 5 bis 2000 ppm Alkalisalze schwacher Säuren enthalten, die im allgemeinen mit nicht in besonderer Weise gereinigten MPG-Estern eingeschleppt werden.

Wie bereits gesagt, gelingt es aus sehr reinem, von Spuren basischer Verunreingungen befreitem MPG-Ester und reinem o-Aminothiophenol in einem unpolaren Lösungsmittel ohne Zusatz von Eisenverbindungen das gewünschte threo-AHMPE mit hoher Ausbeute und hoher Selektivität herzustellen. Das setzt aber aufwendige Reinigungsschritte voraus, z.B. mehrfaches Umkristallisieren oder Umlösen des MPG-Esters z.B. aus wasserhaltigem Isopropanol (siehe Vergleichsbeispiel 2) oder eine sehr intensive Extraktion der Lösung des MPG-Esters mit Wasser und anschließender Trocknung der Lösung. Trotz solcher Maßnahmen gelingt es nicht in jedem Fall zu vermeiden, daß Spuren von Alkalisalzen schwacher Säuren im MPG-Ester oder dessen Lösung verbleiben. Durch die erfindungsgemäße Gegenwart von Eisenverbindungen bei der Reaktion kann das Herstellungsverfahren für threo-AHMPE wesentlich vereinfacht werden, was zur Kostenersparnis und Verringerung von umweltbelastenden Abwassermengen führt.

Eine weitere Verbesserung des Standes der Technik wird erreicht, wenn man zunächst die durch alkalische Spaltung von Benzothiazol erhaltene wäßrige Lösung eines Alkalisalzes von o-Aminothiophenol in Gegenwart einer geringen Menge eines unpolaren Lösungsmittels bis zu einem pH-Wert von 4 bis 6 ansäuert, die dabei sich sehr gut von der wäßrigen Salzlösung abscheidende organische Phase abtrennt und sie nach Extraktion mit Wasser zur Entfernung von restlichem Salz nach Andestillieren bei schwachem Vakuum zur Abtrennung von anhaftendem Wasser und leicht siedenden Verunreinigungen anschließend bei erhöhter Temperatur mit einer Lösung des MPG-Esters im gleichen organischen unpolaren Lösungsmittel in Gegenwart von Eisen zur Umsetzung bringt. Die bei dieser Maßnahmenkombination benötigte Lösungsmittelmenge beträgt maximal 12 % des Volumens des rohen o-Aminothiophenols. Das Ansäuern kann beispielsweise mit Schwefelsäure bis zu einem pH-Wert von 4,5 bis 5,5 und in Gegenwart von Toluol oder Xylol erfolgen. Das Andestillieren kann beispielsweise bei Temperaturen bis zu 50°C erfolgen.

Solche Maßnahmenkombinationen führen zu einer verbesserten Phasentrennung bei der Herstellung von o-Aminothiophenol und ersparen eine zeitraubende und ausbeuteschmälernde Destillation der gesamten, in die Umsetzung mit MPG-Ester einzusetzende Menge an o-Aminothiophenol.

Die folgenden Beispiele erläutern die vorliegende Erfindung.

### Beispiele

Prozentangaben sind Gewichtsprozente, soweit nichts anderes gesagt ist.

### Vergleichsbeispiel 1

### (Einsatz von Alkalisalze schwacher Säuren enthaltendem MPG-Ester, kein Zusatz von Eisenverbindungen)

In 100 ml Xylol wurden 20,9 g 3-(4'-Methoxyphenyl)-glycidsäuremethylester gelöst, der einen Natrium-Gehalt von 440 ppm (davon nur 90 ppm als Natriumchlorid vorliegend) aufwies. Die Lösung wurde zweimal mit jeweils 50 ml Wasser extrahiert und durch Erhitzen bis maximal 145°C Sumpftemperatur restliches Wasser entfernt. In die auf 115°C abgekühlte Lösung wurden unter Rühren 14,4 g eisenfreies o-Aminothiophenol eingetragen und das Gemisch unter weiterem Rühren für 4 Stunden auf 115°C erhitzt. Nach dem Abkühlen auf 70°C ergab eine HPLC-Analyse der klaren Lösung einen Anteil von 86,4 % an der gewünschten threo-Additionsverbindung.

### Beispiel 1

220,9 g 3-(4'-Methoxyphenyl)-glycidsäuremethylester mit einem Gehalt an Natrium von 440 ppm, davon nur 90 ppm als Natriumchlorid, wurden in 100 ml Xylol gelöst. Die Lösung wurde zweimal mit jeweils 50 ml Wasser ausgerührt. Nach Phasentrennung wurde die Lösung unter Auskreisen von restlichem Wasser unter Stickstoff bis 145°C erhitzt. Die erhaltene klare Lösung wurde auf 115°C abgekühlt, 2,0 mg Eisen(III)-chlorid und 14,4 g o-Aminothiophenol zugefügt und das Gemisch noch 4 Stunden unter Rühren bei 115°C gehalten. Nach dem Abkühlen auf 70°C erhielt man 186,0 g einer Lösung mit einem durch HPLC bestimmten Gehalt von 16,6 % des gewünschten threo-AHMPE, entsprechend 92,8 % der Theorie, und 1,04 % erythro-AHMPE, entsprechend 5,8 % der Theorie. Die Ausbeute betrug nach Entfernen des Lösungsmittels, Aufnehmen in Toluol, Kristallisieren, Absaugen und Trocknen 29,2 g eines 96,7 %igen threo-AHMPEs entsprechend 95 % der Theorie. Durch Umlösen in Toluol wurde ein 99,4 %iges threo-AHMPE erhalten.

### Vergleichsbeispiel 2

### (Einsatz von hochreinem MPG-Ester, kein Zusatz von Eisenverbindungen)

In einem 1 l Rührkolben wurden 69,6 g o-Aminothiophenol (98,9 %ig) und 105,2 g 3-(4'-Methoxyphenyl)-glycidsäuremethylester (100 %ig, erhalten durch mehrmalige Umkristallisation aus Isopropanol/Wasser) in 500 ml Toluol vorgelegt und unter Stickstoff auf 115°C erhitzt. Die klare Lösung wurde 4 Stunden bei dieser Temperatur gerührt und danach abgekühlt. Die Ausbeute an Rohlösung betrug 574,7 g, mit einem durch HPLC bestimmten Gehalt an 26,7 % threo-AHMPE, entsprechend 91,8 % der Theorie und 1,7 % erythro-AHMPE, entsprechend 5,8 % der Theorie.

### Beispiele 2 bis 7

Die Beispiele 2 bis 7 bestehen jeweils aus zwei Versuchen, wobei im ersten (a)-Versuch, nicht erfindungsgemäß) die Addition von eisenfreiem o-Aminothiophenol an hochgereinigten MPG-Ester in Xylol unter Zugabe geringer Mengen Natriumacetat oder Natrium-3-(4'-methoxyphenyl)-glycidat durchgeführt wurde. Im jeweils damit vergleichbaren zweiten Versuch (b)-Versuch, erfindungsgemäß) wurde außer der Zugabe von Natriumacetat oder Natrium-3-(4'-methoxyphenyl)-glycidat o-Aminothiophenol mit einem Gehalt von 5 ppm Eisen in Form von Eisen(III)-chlorid verwendet.

Durchführung der Versuche:
In 100 ml Xylol wurden jeweils 14,4 g o-Aminothiophenol (96 %ig), 20,9 g 3-(4'-Methoxyphenyl)-glycidsäuremethylester (umkristallisiert, 100 %ig) und der jeweils angegebene Natriumsalz-Zusatz unter Stickstoff bis 115°C erhitzt und 4 Stunden bei dieser Temperatur gerührt. Nach dem Abkühlen der Lösung wurde das Ergebnis durch eine HPLC-Analyse ermittelt.

Bei den Beispielen 2 bis 5 wurde Natriumacetat und bei den Beispielen 6 und 7 Natrium-3-(4'-methoxyphenyl)-glycidat zugegeben.

Die Einzelheiten und Ergebnisse sind aus Tabelle 1 ersichtlich.

Man ersieht aus Tabelle 1, daß die Ausbeuten threo + erythro-AHMPE und die Anteile des gewünschten threo-AHMPE am gesamten Additionsprodukt bei Zusatz von Eisensalzen stets deutlich höher sind als bei der Abwesenheit von Eisensalzen.

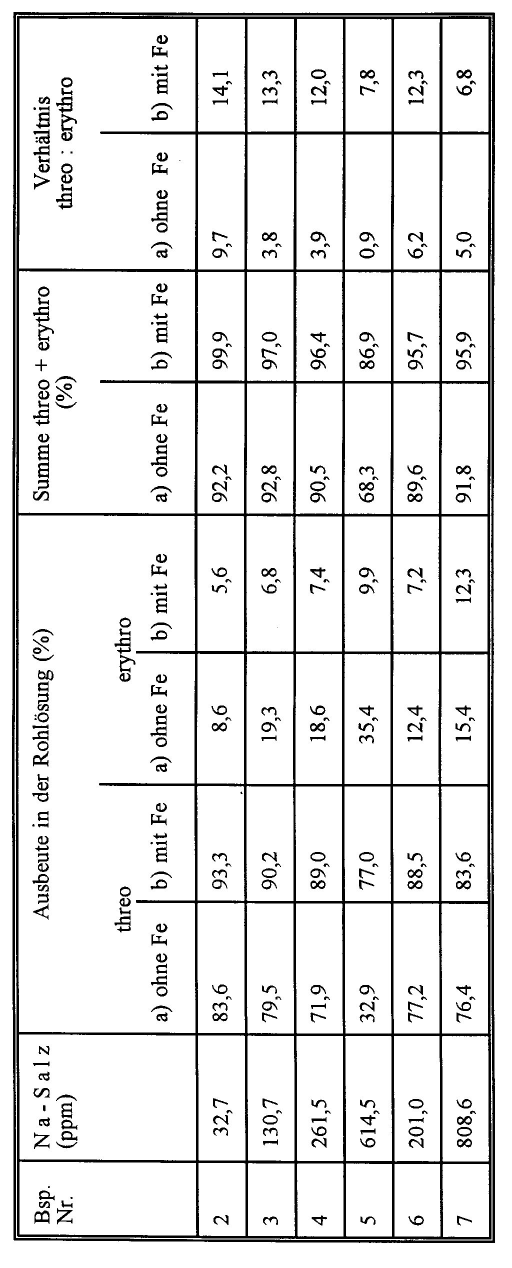

### Beispiel 8

270,1 g einer 30 %igen Natriummethylatlösung in Methanol und zusätzlich 50 ml Methanol wurden in einem Rührkolben vorgelegt und unter Rühren auf 0°C gekühlt. Aus zwei Tropftrichtern wurden getrennt voneinander aber gleichzeitig innerhalb von 2 Stunden 137,5 g Anisaldehyd (99 %ig) und 115,1 g Chloressigsäuremethylester (99 %ig) bei konstant 0°C unter Rühren zugetropft. Das Reaktionsgemisch wurde anschließend 4 Stunden bei 0°C nachgerührt. Danach tropfte man unter weiterem Kühlen 35 ml Eisessig zu, wobei die Temperatur des Gemisches +5°C nicht überstieg. Hierauf gab man 800 ml Toluol zu, rührte das Gemisch kräftig durch und fügte anschließend 500 ml Wasser zu. Man rührte das Gemisch kräftig durch, trennte die Phasen, wusch die Toluolphase dreimal mit jeweils 100 ml Wasser choridfrei und führte ohne Verzögerung eine Vakuumdestillation bei 45°C und 95 mbar zur azeotropen Entwässerung der Lösung durch. Man erhielt 751,3 g Lösung mit einem Gehalt von 23,9 % des gewünschten 3-(4'-Methoxyphenyl)-glycidsäuremethylesters, entsprechend 86,2 % der Theorie.

435,9 g der so erhaltenen toluolischen Lösung des Glycidesters wurden unter Stickstoff-Begasung auf 115°C erhitzt. Bei dieser Temperatur wurden innerhalb von 10 Minuten 73,2 g o-Aminothiophenol (erhalten wie in Beispiel 9 beschrieben, aber ohne Entfernung des Lösungsmittels, jedoch mit kurzem Andestillieren zur Entfernung von Wasser), das 5 ppm Eisen in Form von Eisen(III)-chlorid enthielt, zugetropft. Das Gemisch wurde danach 4 Stunden am Rückfluß erhitzt und gleichzeitig Wasser ausgekreist. Man erhielt 60 ml einer Toluol-Lösung mit einem Gehalt von 26,2 % threo-AHMPE, entsprechend 90,6 % der Theorie. Die Lösung enthielt außerdem 1,8 % erythro-AHMPE, entsprechend 6,4 % der Theorie. Die Toluol-Lösung wurde auf 50°C abgekühlt, mit Impfkristallen von threo-AHMPE versetzt und auf 5°C abgekühlt. Nach vollständiger Kristallisation wurde das Gemisch bei 5°C abgesaugt und der Rückstand mit 75 ml gekühltem Toluol und danach mit einer Lösung aus 50 ml Methanol und 50 ml Wasser gewaschen. Nach dem Trocknen erhielt man 140,5 g farblose Kristalle, was einer Ausbeute von 84,3 % der Theorie an threo-AHMPE entspricht.

### Beispiel 9

### (vorteilhafte Isolierung von o-Aminothiophenol aus einem Reaktionsgemisch)

1 kg o-Aminothiophenol-Natriumsalz-Lösung (technisch, ca. 25 %ig), erhalten durch Spaltung von Benzothiazol mit Natronlauge bei erhöhter Temperatur, wurden unter Stickstoff in einem Rührkolben mit Bodenventil vorgelegt. Unter Rühren tropfte man aus einem Tropftrichter 310 g 48 %ige wäßrige Schwefelsäure zu bis zu einem pH-Wert von 4,8 und unter Kontrolle der Temperatur, die 50°C nicht überstieg. Man fügte nun 100 ml Toluol zu, rührte kräftig durch und erhielt eine schnelle und gute Trennung zwischen der organischen und der wäßrigen Phase. Die untere, wäßrige, salzhaltige Phase wurde durch das Bodenventil abgelassen. Die organische Phase wurde nach Zugabe von 100 ml Wasser kräftig durchgerührt und so von restlichem gelöstem Salz befreit. Anschließend wurde sie durch das Bodenventil abgelassen. Man erhielt so 282 g einer hellgelben o-Aminothiophenol-Lösung in Toluol. Nach Andestillieren zum Entfernen des Lösungsmittels bei 55 bis 58 mbar und bis zu einer Sumpftemperatur von 122°C erhielt man 198 g o-Aminothiophenol folgender Zusammensetzung (GC):

| | |
|---|---|
| o-Aminothiophenol | 97,3 % |
| Toluol | 0,8 % |
| Anilin | 0,2 % |
| 2-Methylanilin | unter 0,1 % |
| isomeres Aminothiophenol | 0,1 % |
| Methylmercaptoanilin | 0,2 % |
| 2,2'-Diaminodiphenyldisulfid | 0,9 % |
| Benzothiazol | unter 0,1 % |

Solches o-Aminothiophenol erwies sich als besonders geeignet für die Umsetzung mit 3-(4'-Methoxyphenyl)-glycidsäuremethylester in Gegenwart von Eisenverbindungen.

## Patentansprüche

1. Verfahren zur Herstellung von (2RS, 3RS)-3-(2'-Aminophenylthio)-2-hydroxy-3-(4''-methoxyphenyl)-propionsäuremethylester durch Addition von o-Aminothiophenol an 3-(4'-Methoxyphenyl)-glycidylsäuremethylester, dadurch gekennzeichnet, daß man die Reaktion bei Anwesenheit von Alkali-salzen schwacher Säuren in Gegenwart katalytischer Mengen von Eisenverbindungen durchführt.

2. Verfahren nach Anspruch 1, dadurch gekennzeichnet, daß man Eisenverbindungen einsetzt, die 2- und/oder 3-wertiges Eisen enthalten.

3. Verfahren nach Ansprüchen 1 und 2, dadurch gekennzeichnet, daß man Eisen(III)-sulfat-pentahydrat, Eisen(II)-sulfat, Eisen(III)-chlorid, Eisen(II)-chlorid, Eisensalze organischer Säuren und/oder komplexe Eisenverbindungen einsetzt.

4. Verfahren nach Ansprüchen 1 bis 3, dadurch gekennzeichnet, daß man 1 bis 200 ppm Eisenverbindungen, gerechnet als Eisen und bezogen auf o-Aminothiophenol, einsetzt.

5. Verfahren nach Ansprüchen 1 bis 4, dadurch gekennzeichnet, daß man die Reaktion in einem unpolaren Lösungsmittel durchführt.

6. Verfahren nach Ansprüchen 1 bis 5, dadurch gekennzeichnet, daß man die Reaktion bei 80 bis 150°C durchführt.

7. Verfahren nach Ansprüchen 1 bis 6, dadurch gekennzeichnet, daß das Reaktionsgemisch 5 bis 2000 ppm Alkalisalze schwacher Säuren enthält.

8. Verfahren nach Ansprüchen 1 bis 7, dadurch gekennzeichnet, daß man o-Aminothiophenol einsetzt, das erhalten wurde, indem man die durch alkalische Spaltung von Benzothiazol erhaltene wäßrige Lösung eines Alkalisalzes von o-Aminothiophenol in Gegenwart einer geringen Menge eines unpolaren Lösungsmittels bis zu einem pH-Wert von 4 bis 6 ansäuert, die sich abscheidende organische Phase abtrennt und sie nach Extraktion mit Wasser und Andestillieren bei schwachem Vakuum von restlichen Salzen, anhaftendem Wasser und leicht siedenden Verunreinigungen befreit.
